# EUROPÄISCHE PATENTSCHRIFT

(11) **EP 0 913 132 B1**
(45) Veröffentlichungstag und Bekanntmachung des Hinweises auf die Patenterteilung: **11.02.2004**
(21) Anmeldenummer: 97810802.5
(22) Anmeldetag: 28.10.1997
(51) Int. Cl.: A61F 2/38

(54) **Kniegelenkprothese**
Knee prosthesis
Prothèse de genou

(43) Veröffentlichungstag der Anmeldung: 06.05.1999
(73) Patentinhaber: Centerpulse Orthopedics Ltd., 6340 Baar (CH)
(72) Erfinder: Livet, Pascal, 8400 Winterhur (CH); Frei, Heribert, 8200 Schaffhausen (CH); Brack, René, 6330 Cham (CH)
(74) Vertreter: Manitz, Finsterwald & Partner Gbr

(56) Entgegenhaltungen:
- EP-A- 0 724 868
- DE-A- 4 434 806
- GB-A- 2 296 443
- US-A- 5 395 401
- US-A- 5 405 395

## Beschreibung

Die Erfindung betrifft eine Kniegelenkprothese.

Kniegelenkprothesen stehen heutzutage in einer grossen Anzahl verschiedener Ausführungsformen zur Verfügung, wobei ihre Funktionsprinzipien sich oft stark voneinander unterscheiden. Dies hängt - unter anderem - damit zusammen, ob und gegebenenfalls welche Bänder (z.B. Kreuzbänder, Seitenbänder) bei dem jeweiligen Patienten, dem die Prothese implantiert werden soll, noch vorhanden bzw. funktionstüchtig sind.

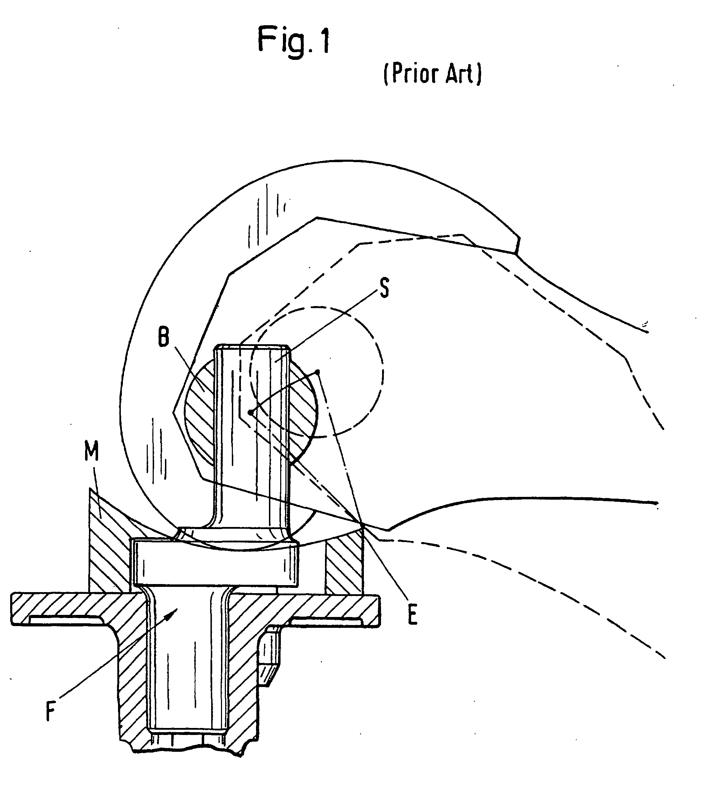

Eine derartige Kniegelenkprothese ist beispielsweise in der EP-A-0,724,868 offenbart. Die dort beschriebene Kniegelenkprothese umfasst ein Tibiateil, welches in der Tibia verankert wird und welches eine Tibialagerfläche aufweist, auf der ein Lagerkörper in Form eines Meniskusteils gleitend verschiebbar angeordnet ist. Die Bahn, entlang der das Meniskusteil geführt wird, ist in einigen Ausführungsbeispielen (z.B. Figs.1a,1b,1c) auf die anteriore/posteriore Richtung beschränkt. Zu diesem Zweck ist das Tibiateil mit einem Führungsteil oder Führungsstück versehen, welches in ein entsprechendes Führungsteil (Langloch bzw. Schwalbenschwanz-Nut) in dem Meniskusteil eingreift, wodurch das Meniskusteil nur in anteriorer/posteriorer Richtung verschiebbar ist.

Das Führungsstück, das am Tibiateil vorgesehen ist, weist einen stabförmigen Fortsatz auf, der in proximaler Richtung weist, also in Richtung auf das Femurteil zu. Dieser stabförmige Fortsatz erstreckt sich in einer Richtung parallel zur Längsachse des Tibiateils. Zur Kopplung dieses Fortsatzes und damit der am Tibiateil vorgesehenen Führungsstücks mit dem Femurteil ist ein Kupplungsorgan in Form eines zylindrischen Bolzens vorgesehen, der eine senkrecht zur Längsachse des Bolzens verlaufende Durchgangsbohrung aufweist.

Das Femurteil weist zwei Kondylen auf, die sich auf den entsprechenden Lagerschalen (Lagerflächen) des Lagerkörpers bewegen können. Das Femurteil weist ferner zwei Seitenwände auf, in denen jeweils eine die Seitenwand durchbrechende, zylindrische Ausnehmung vorgesehen ist.

In diese Ausnehmungen in den Seitenwänden wird der zuvor beschriebene zylindrische Bolzen mit seiner senkrecht zur Längsachse des Bolzens verlaufenden Durchgangsbohrung eingesetzt und der stabförmige Fortsatz des Führungsstücks wird durch die zylindrische Bohrung des Bolzens hindurchgeführt. Der Bolzen wirkt somit als Kupplungsorgan, weil er bei der Flexions-bzw. bei der Extensionsbewegung des Knies das Femurteil mit dem Fortsatz am Führungsstück koppelt. Bei der Extension bzw. bei der Flexion des Knies gleitet der zylindrische Bolzen an dem Fortsatz des Führungsstücks entlang aufwärts (Extension) bzw. abwärts (Flexion). Gleichzeitig verschiebt sich das Meniskusteil in anteriorer (Extension) bzw. posteriorer Richtung (Flexion).

Die in der EP-A-0,724,868 beschriebene Kniegelenkprothese ist vom Prinzip her absolut funktionstüchtig. Allerdings ist das Zusammensetzen der Prothese im Operationssaal für den Operateur nicht ganz einfach, weil nach dem Fixieren des Tibiateils in der Tibia und des Femurteils im Femur der stabförmige Fortsatz des Führungsstücks durch die Bohrung in dem zylindrischen Bolzen geführt werden muss, also praktisch eine Art "Einfädelvorgang" durchgeführt werden muss. Dies kann dem Operateur unter Umständen Schwierigkeiten bereiten, da ja das Tibiateil und das Femurteil bereits an den entsprechenden Knochen fixiert sind.

Darüberhinaus kann bei der in der EP-A-0,724,868 beschriebene Prothese aber auch noch der nachfolgende, anhand von Fig. 1 beschriebene Fall auftreten. Bei der Flexion bewegt sich der zylindrische Bolzen B an dem stabförmigen Fortsatz S des Führungsstücks F entlang abwärts, während das Meniskusteil M sich in posteriorer Richtung bewegt. Nun kann der Fall auftreten, dass der Femur auf dem hinteren Ende E des Meniskusteils M aufliegt, obwohl noch keine vollständige Beugung erfolgt ist. Würde man nun eine weitere Beugung versuchen durchzuführen, so würde das Drehzentrum durch das hintere Ende E des Meniskusteils M gebildet werden müssen. Dies würde bedeuten, dass sich bei einer weiteren Beugung der zylindrische Bolzen B in die strichliert angedeutete Position bewegen müsste. Dies kann aber deshalb nicht erfolgen, weil der Bolzen B ja nur an dem Fortsatz S des Führungsstücks F entlang aufwärts und abwärts bewegt werden kann (und nicht auf einer Kreisbahn, wie er es müsste). Eine Blockierung der Pothese ist die Folge, welche eine weitere Flexion verhindert. In schlimmeren Fällen, wenn die Kräfte genügend gross sind, kann sogar eine Lockerung des Femurteils der Prothese die Folge sein.

Zum Stand der Technik wird außendem auf die DE 44 34 806 A1 verwiesen, die Kniegelenkprothesen mit einem Kopplungszapfen zwischen Femurteil und Tibiateil beschreibt, wobei das Femurteil aber jeweils derart ausgestaltet ist, dass es auch ohne Kopplungzapfen vall funktions Fähig ist.

Es ist Aufgabe der Erfindung, eine Kniegelenkprothese des genannten Typs vorzuschlagen, bei denen die vorstehend beschriebenen Probleme vermieden werden, bei der also insbesondere eine problemlose Flexion möglich ist. Darüberhinaus soll die Prothese während der Operation einfach zusammenzusetzen sein, also speziell dann, wenn das Tibiateil und das Femurteil bereits an den zugehörigen Knochen fixiert sind. Die Prothese soll in hohem Masse zuverlässig sein und sie soll vor allen Dingen auch für Patienten geeignet sein, bei denen sowohl die Kreuzbänder als auch die Seitenbänder entweder nicht mehr vorhanden oder nicht mehr funktionstüchtig sind.

Diese Aufgabe wird durch die erfindungsgemässe Kniegelenkprothese gelöst. Die erfindungsgemässe Kniegelenkprothese umfasst ein Tibiateil, welches eine Tibialagerfläche aufweist. Ferner umfasst sie einen Lagerkörper (Meniskusteil), der auf der Tibialagerfläche gleitend verschiebbar gelagert ist und der auf seiner der Tibialagerfläche abgewandten Seite Lagerschalen aufweist. Weiterhin umfasst sie ein Femurteil, welches auf den Lagerschalen des Lagerkörpers beweglich angeordnet ist, sowie ein relativ zum Tibiateil drehfest angeordnetes Führungsstück, welches lediglich eine Verschiebung des Lagerkörpers in sagittaler Richtung relativ zur Tibialagerfläche gestattet. Schliesslich umfasst die erfindungsgemässe Kniegelenkprothese noch ein Kupplungsorgan, welches sowohl mit dem Führungsstück als auch mit dem Femurteil zusammenwirkt und eine Drehung des Femurteils auf dem Lagerkörper erlaubt. Dieses Kupplungsorgan weist einen drehbar gelagerten Zapfen auf, der sich in Richtung der Längsachse des Tibiateils bzw. einer hierzu parallelen Drehachse erstreckt oder in Richtung einer relativ zur Längsachse des Tibiateils bzw. einer hierzu parallelen Drehachse geneigten Achse. Mit einer derartigen Prothese werden die eingangs beschriebenen Probleme bei der Flexion vermieden, weil das Drehzentrum nicht an eine feste Aufwärts- bzw. Abwärtsrichtung gebunden ist. Darüberhinaus ist eine derartige Kniegelenkprothese während der Operation einfach zusammenzusetzen, und sie ist insbesondere auch für Patienten geeignet, bei denen sowohl die Kreuzbänder als auch die Seitenbänder entweder nicht mehr vorhanden oder nicht mehr funktionstüchtig sind.

Bei der erfindungsgemässen Kniegelenkprothese weist der als Kupplungsorgan dienende Zapfen ferner an seinem dem Femurteil zugewandten Ende ein Stabilisierungsstück auf und das Femurteil entsprechende mit dem Stabilisierungsstück zusammenwirkende Stabilisierungsflächen. Auf diese Weise wird eine Varus/Valgus-Stabilisierung erreicht, sodass ein seitliches Verkippen von Femurteil und Tibiateil relativ zueinander verhindert wird.

Der als Kupplungsorgan dienende Zapfen wird von einer im Führungsstück vorgesehenen Bohrung aufgenommen, in welcher er drehbar gelagert ist. Dabei kann beispielsweise das Führungsstück direkt am Tibiateil angeformt sein.

Bei einem weiteren Ausführungsbeispiel sind sowohl das Führungsstück als auch das Kupplungsorgan beide als separate Zapfen ausgebildet, wobei sowohl der als Führungsstück dienende Zapfen als auch das Tibiateil Mittel zur drehfesten Anordnung des als Führungsstück dienenden Zapfens im Tibiateil aufweisen. Der als Führungsstück dienende Zapfen wird somit drehfest in dem Tibiateil verankert, während der als Kupplungsorgan dienende Zapfen drehbar gelagert ist. Die Zapfen sind somit getrennt herstellbar, wodurch insbesondere die Herstellung des Führungsstücks einfach ist, aber auch die Herstellung des Tibiateils, insbesondere der Tibialagerfläche, einfach ist.

Bei einem weiteren Ausführungsbeispiel weist das Tibiateil einen Verankerungsabschnitt auf, der mit einem separaten Verankerungsschaft verbindbar ist, bei wiederum einem weiteren Ausführungsbeispiel weist das Femurteil einen Verankerungsabschnitt auf, der mit einem separaten Verankerungsschaft verbindbar ist. Dadurch ist es möglich, einen Verankerungsschaft zu verwenden, der eine speziell für den jeweiligen Patienten optimale Länge aufweist. Die definitive Entscheidung für den optimalen Verankerungsschaft kann sogar noch während einer Operation getroffen werden, wenn der Operateur die tatsächlichen anatomischen Verhältnisse genau erkennen kann.

Im folgenden wird die Erfindung anhand der Zeichnung näher erläutert. Es zeigen, teilweise in schematischer Darstellung und/oder im Schnitt:
- Fig. 1: die aus der EP-A-0,724,868 bekannte Prothese in verschiedenen Positionen,
- Fig. 2: eine Explosionsdarstellung eines Ausführungsbeispiels einer erfindungsgemässen Kniegelenkprothese,
- Fig. 3: eine Explosionsdarstellung eines Ausführungsbeispiels eines als Kupplungsorgan dienenden Zapfens, des Lagerkörpers, und von verschiedenen Ausführungsbeispielen von Zapfen, die als Führungsstück dienen,
- Fig. 4: eine perspektivische Ansicht der Zapfen aus Fig. 2, die das Führungsstück bilden, von schräg unten,
- Fig. 5: ein Ausführungsbeispiel eines Tibiateils, in welchem ein als Führungsstück dienender Zapfen mittels einer Schraubverbindung verankert ist
- Fig. 6: ein weiteres Ausführungsbeispiel eines Tibiateils, in welchem ein weiteres Ausführungsbeispiel eines als Führungsstück dienenden Zapfens verankert ist
und
- Fig. 7: ein weiteres Ausführungsbeispiel einer erfindungsgemässen Kniegelenkprothese (ohne Femurteil).

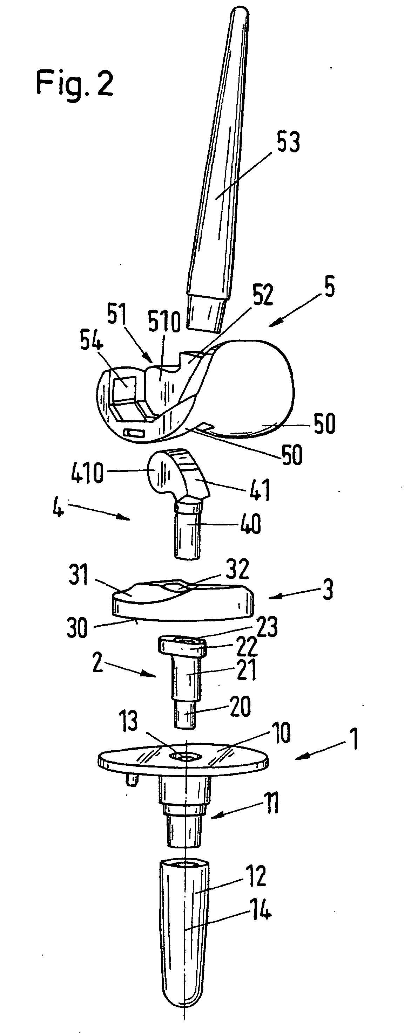

In der Explosionsdarstellung eines Ausführungsbeispiels einer erfindungsgemässen Kniegelenkprothese gemäss Fig. 2 erkennt man die wesentlichen Teile der Kniegelenkprothese. Im wesentlichen sind dies ein Tibiateil 1, ein Zapfen 2, ein Lagerkörper 3, ein weiterer Zapfen 4, sowie ein Femurteil 5.

Etwas detaillierter betrachtet erkennt man ein (üblicherweise metallisches) Tibiateil 1 mit einer Tibialagerfläche 10, die in dem gezeigten Ausführungsbeispiel als ebene Fläche ausgebildet ist, die aber vom Prinzip her auch gekrümmt ausgebildet sein kann. Das Tibiateil 1 weist ferner einen Verankerungsabschnitt 11 auf, der mit einem Verankerungsschaft 12 verbindbar ist, zum Beispiel mittels einer konischen Verbindung. In dem Tibiateil 1 ist ferner eine als Sackloch ausgebildete Bohrung 13 zu erkennen.

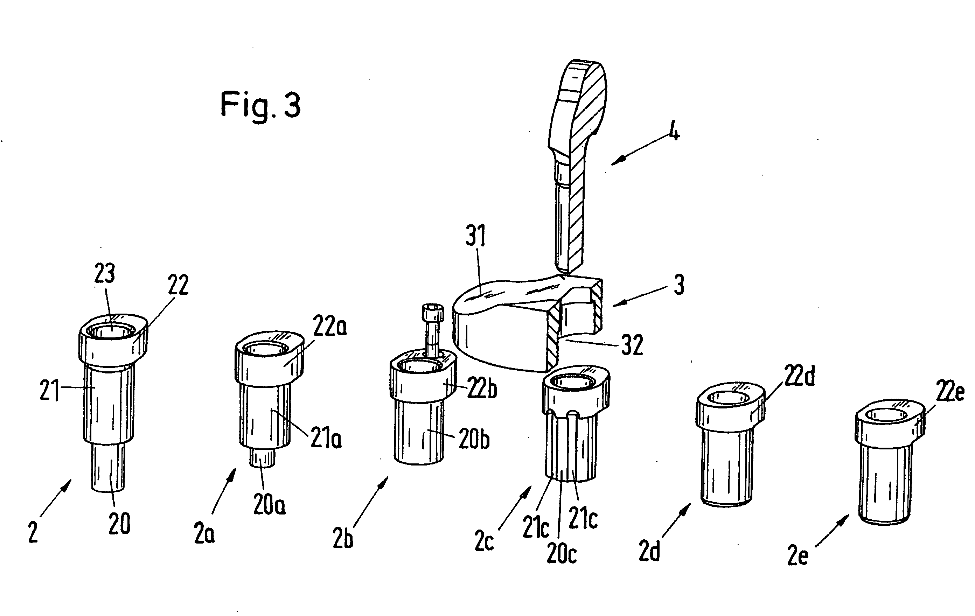

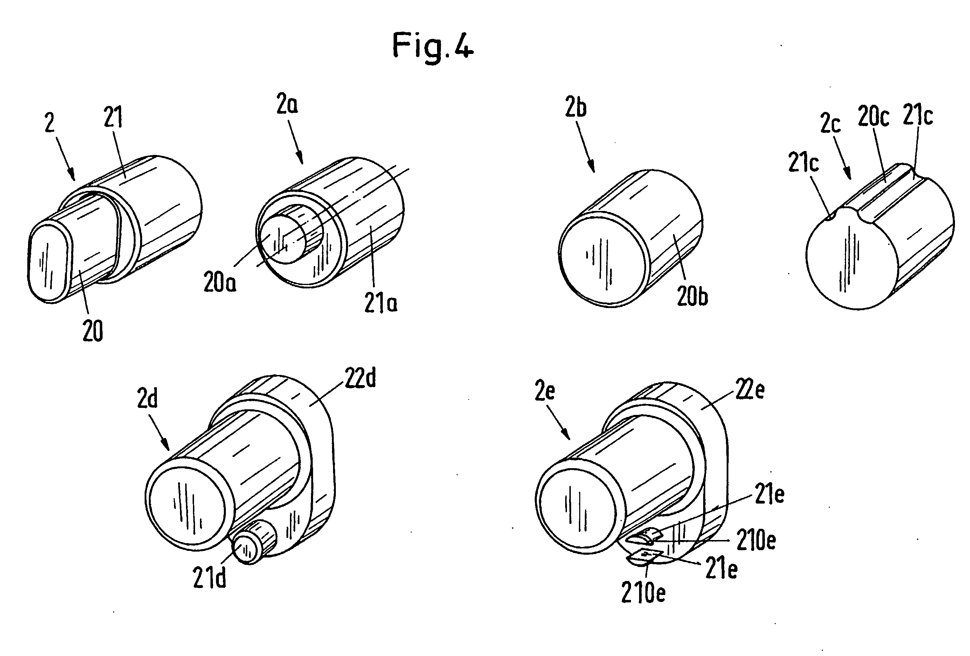

In die als Sackloch ausgebildete Bohrung 13 des Tibiateils 1 ist der als Führungsstück ausgebildete Zapfen 2 einsetzbar. Der Zapfen 2 (typischerweise metallisch) weist hier drei Bereiche auf, einen unteren Bereich 20 einen mittleren Bereich 21, und einen oberen Bereich 22. Bei dem in Fig. 2 gezeigten Ausführungsbeispiel ist die äussere Gestalt des unteren Bereichs 20 oval ausgebildet (wird anhand von Fig. 3 bzw. Fig. 4 noch genauer gezeigt). Damit der Zapfen 2 in die Bohrung 13 des Tibiateils 1 eingesetzt werden kann, muss der entsprechende Bereich der Bohrung 13 bei dem hier beschriebenen Ausführungsbeispiel ebenfalls eine ovale Gestalt aufweisen (die z.B. durch Fräsen hergestellt werden kann). Beim Einsetzen des Zapfens 2 werden der untere Bereich 20 und der mittlere Bereich 21 des Zapfens von der Bohrung 13 im Tibiateil 1 aufgenommen. Durch den unteren, ovalen Bereich 20, der von dementsprechende ovalen Bereich der Bohrung 13 aufgenommen wird, ist der Zapfen 2 im Tibiateil 1 drehfest angeordnet, er kann sich also nach dem Einsetzen relativ zum Tibiateil 1 nicht verdrehen. Der obere Bereich 22 des Zapfens 2 liegt auf der Tibialagerfläche 10 auf. Die Bohrung 13 im Tibiateil 1 ist dabei so angeordnet, dass sich bei in die Bohrung 13 eingebrachtem Zapfen 2 der obere Bereich 22 des Zapfens 2 in sagittaler Richtung erstreckt. Schliesslich erkennt man noch, dass der Zapfen 2 ebenfalls eine als Sackloch ausgebildete Bohrung 23 aufweist. Es ist unmittelbar einleuchtend, dass anstelle des unteren Bereichs auch der mittlere Bereich 21 und der entsprechende Bereich der Bohrung im Tibiateil 1 oval ausgebildet sein können, sodass die drehfeste Anordnung durch den mittleren Bereich des Zapfens 2 und den zugehörigen Teil der Bohrung im Tibiateil 1 bewirkt werden. Weiter unten werden sogar noch Fälle erläutert, wo mit Hilfe des oberen Bereichs 22 (und speziellen Mitteln) die drehfeste Anordnung des Zapfens 2 im Tibiateil 1 bewirkt wird.

Der als Meniskusteil dienende Lagerkörper 3, üblicherweise aus einem Kunststoff wie Polyethylen hergestellt, weist auf seiner zum Tibiateil 1 weisenden Unterseite eine zur Tibialagerfläche 10 korrespondierende (hier: ebene) Gleitfläche 30 auf, sodass der Lagerkörper 3 auf der Tibialagerfläche 10 gleiten kann. Ferner ist der Lagerkörper 2 mit zwei dem Femurteil zugewandten Lagerschalen 31 versehen, die hier integraler Bestandteil des Lagerkörpers 3 sind (der Lagerkörper ist bei dem beschriebenen Ausführungsbeispiel also einstückig), die aber auch als separat herstellbare Lagerschalen ausgebildet sein können, die in einen brillenartig ausgebildeten Lagerkörper eingesetzt werden können und dann zusammen mit diesem das Meniskusteil bilden. Der Lagerkörper weist ferner ein Langloch 32 auf, dessen Breite so bemessen ist, dass es den oberen Bereich 22 des Zapfens 2 aufnehmen kann und ggf. ein geringes Spiel hat. Die Länge des Langlochs 32 ist so bemessen, dass das Langloch 32 um ein vorgegebenes Mass länger ist als der obere Bereich 22 des Zapfens 2, wobei dieses vorgegebene Mass so bemessen ist, dass sich der Lagerkörper 3 bei der Flexion um ein vorgebbares Mass in sagittaler Richtung verschieben kann. Der Zapfen 2 wirkt somit als Führungsteil bezüglich des Lagerkörpers 3 und lässt insbesondere nur eine Verschiebung des Lagerkörpers 3 auf der Tibialagerfläche 10 in sagittaler Richtung nach hinten zu.

Ein weiterer Zapfen 4 weist einen kreiszylindrischen Abschnitt 40 auf und ein daran anschliessendes Stabilisierungsstück 41 auf. Das Stabilisierungsstück 41 wiederum zeichnet sich insbesondere durch zwei Seitenwände 410 aus, von denen in Fig. 2 nur eine zu erkennen ist. Diese Seitenwände 410 des Stabilisierungsstücks 41 des Zapfens 4 wirken mit entsprechenden Stabilisierungsflächen des Femurteils 5 zusammen und bewirken eine Varus/Valgus-Stabilisierung des Femurteils 5 relativ zum Tibiateil 1.

Das Femurteil 5 weist zwei Kondylen 50 auf, die mit den Lagerschalen 31 des Lagerkörpers 3 zusammenwirken. Die Kondylen 50 weisen dabei unterschiedliche Krümmungsradien auf, zudem sind auch die Drehzentren bei der Flexion unterschiedlich. In der Extension sind die Kondylen 50 in hohem Masse kongruent zu den Lagerschalen 31 und der Kontakt zwischen den Kondylen 50 und den Lagerschalen 31 ist grossflächig, in der Flexion hingegen ist der Kontakt zwischen den Kondylen 50 und den Lagerschalen 31 nur auf eine geringe Fläche verteilt oder gar linienförmig, weil der Krümmungsradius in dem Bereich der Kondylen 50, der bei der Flexion im Kontakt mit den Lagerschalen 31 steht, deutlich kleiner ist als der Krümmungsradius der Lagerschalen 31.

Das Femurteil 5 weist ferner einen Stabilisierungskasten 51 auf, der insbesondere zwei seitliche Stabilisierungsflächen 510 aufweist, von denen in Fig. 2 aufgrund der gewählten Darstellung nur eine zu erkennen ist. Diese Stabilisierungsflächen 510 wirken mit den Seitenwänden 410 des Stabilisierungsstücks 41 des Zapfens 4 zusammen und bewirken die bereits erwähnte Varus/Valgus-Stabilisierung des Femurteils 5 gegenüber dem Tibiateil 1.

Weiterhin weist das Femurteil 5 einen Verankerungsabschnitt 52 auf, der mit einem Verankerungsschaft 53 verbindbar ist, zum Beispiel mittels einer konischen Verbindung. Schliesslich weist das Femurteil 5 auf der Rückseite der Kondylen noch einige Zementtaschen 54 auf, welche zur Aufnahme von Knochenzement dienen, wie er bei der Befestigung der Femurteils 5 am Femur üblicherweise zum Einsatz kommt.

Das Zusammensetzen des in Fig. 2 gezeigten Ausführungsbeispiels der erfindungsgemässen Kniegelenkprothese erfolgt nun wie folgt. Nachdem die Tibia und der Femur des Patienten vorbereitet worden sind, wird der jeweils gewünschte Verankerungsschaft 12 mit dem Verankerungsabschnitt 11 verbunden. Dadurch ist das Tibiateil 1 als solches praktisch komplett. Das Tibiateil 1 kann nun an der Tibia in bekannter Weise befestigt werden.

Entsprechend wird der Verankerungsschaft 53 mit dem Verankerungsabschnitt 52 verbunden, wodurch das Femurteil 5 komplettiert ist. Das Femurteil 5 wird nun in bekannter Weise am Femur befestigt.

Die "modulare" Gestaltung der Prothese, also die Tatsache, dass praktisch noch im Operationssaal der jeweils optimale Verankerungsschaft für das Tibiateil und das Femurteil ausgewählt werden kann, gewährleistet eine Versorgung des Patienten mit einer praktisch "massgeschneiderten" Prothese. Grundsätzlich könnten das Tibiateil und das Femurteil aber auch bereits als integrales Teil vorliegen, also die Verankerungsschäfte integral mit dem jeweiligen Prothesenteil verbunden sein. Dies würde allerdings entweder die Flexibilität des Operateurs bei der Auswahl einschränken, oder es müsste einfach eine genügend grosse Auswahl an Prothesenteilen bei der Operation zur Verfügung stehen. Die "modulare" Gestaltung der Prothese verringert erheblich die Anzahl der Teile, die zur Verfügung stehen müssen, um eine grosse Auswahlmöglichkeit zu haben und letzlich die Versorgung des Patienten mit einer für ihn optimale Prothese zu gewährleisten.

Das Tibiateil 1 weist bei der bisher beschriebenen Zusammensetzung nun aber noch kein Führungsstück für den Lagerkörper 3 (Meniskusteil) auf. Zu diesem Zweck wird jetzt der Zapfen 2 in die als Sackloch ausgebildete Bohrung 13 des Tibiateils 1 eingebracht und von dieser Bohrung 13 drehfest aufgenommen (wie oben erläutert). Grundsätzlich könnte aber auch das Führungsstück direkt am Tibiateil angeformt sein (es könnte also integraler Bestandteil des Tibiateils sein), die Herstellung des Tibiateils wird dadurch jedoch aufwendiger.

Sodann wird der Lagerkörper 3 - das Meniskusteil - eingebracht, indem der obere Bereich 22 des Zapfens 2 in das Langloch 32 des Lagerkörpers 3 eingeführt wird. Anschliessend wird der kreiszylindrische Bereich 40 des Zapfens 4 in die als Sackloch ausgebildete kreiszylindrische Bohrung 23 des Zapfens 3 eingebracht. Dies alles sind Vorgänge, die von dem Operateur bei der Implantation auf ausserordentlich einfache Weise vorgenommen werden können.

Nun müssen noch das Tibiateil 1 und das Femurteil 5 gekoppelt werden. Dieser Kopplungsvorgang wird nun erheblich erleichtert dadurch, dass sich der Zapfen 4, das Kupplungsorgan, in Richtung der Längsachse 14 des Tibiateils 1, bzw. wenn die Bohrung 13 nicht zentral im Tibiateil 1 angeordnet ist, in Richtung einer zur Längsachse 14 des Tibiateils parallelen Achse. Dadurch ergibt sich kein aufwendiger Einfädelvorgang, sondern Femur und Tibia müssen lediglich so bewegt werden, dass der Zapfen 4 in den Stabilisierungskasten 51 des Femurteils hineingleitet, was ein vergleichsweise einfach durchzuführender, wenig aufwendiger Vorgang ist. Somit sind Tibiateil 1 und Femurteil 5 der Prothese gekoppelt und der reine Implantationsvorgang ist praktisch abgeschlossen.

Auf die Gestaltung der Kondylen 50 des Femurteils 5 und der Lagerschalen 31 des Lagerkörpers 31 ist bereits hingewiesen worden. In der Extension (z.B. beim Stehen) wird die Prothese üblicherweise durch grosse Kräfte belastet (das Körpergewicht lastet ja beim Stehen auf der Prothese). Zwar werden die grossen Kräfte durch die hohe Kongruenz bei der Extension auf eine grosse Fläche verteilt, was die Flächenpressung verringert. Aufgrund der grossen Kräfte und der hohen Kongruenz der gewölbten Flächen ist jedoch in der Extension eine Drehung des Femurteils 5 relativ zum Tibiateil 1 praktisch nicht möglich.

Erfolgt nun eine Beugung, so bewegt sich der Lagerkörper 3 in sagittaler, posteriorer Richtung, was auch dem natürlichen Ablauf beim Beugen des Knies entspricht. Allerdings wird die Bewegung des Lagerkörpers 3 in posteriorer Richtung durch das Langloch 32 begrenzt. In der Flexion, speziell in einem Zustand starker Beugung, ist die Kongruenz jedoch gering bis hin zu einem linienförmigen Kontakt, weil der Krümmungsradius der Kondylen 50 hier deutlich geringer ist als der Krümmungsradius der Lagerschalen 31 des Lagerkörpers 3. Femurteil 5 und Tibiateil 1 können in einem solchen Zustand gegeneinander verdreht werden, da der Zapfen 4 ja im Zapfen 2 drehbar gelagert ist.

Erfolgt nun wieder eine Streckung, so gleitet der Lagerkörper 3 auf der Tibialagerfläche wieder in sagittaler, diesmal aber anteriorer Richtung. Diese Bewegung des Lagerkörpers 3 wird ebenfalls durch das Langloch 32 begrenzt. Im Zustand der Extension ist nun die Kontaktfläche wieder verhältnismässig gross, weil der Krümmungsradius der Kondylen 50 in der Extension dem Krümmungsradius der Lagerschalen 31 entspricht, somit eine hohe Kongruenz vorliegt. Wie bereits beschrieben, ist in diesem Zustand eine Drehung des Femurteils 5 relativ zum Tibiateil 1 praktisch nicht möglich.

Somit weist die Kniegelenkprothese ein Verhalten auf, bei welchem in der Extension keine Rotation zwischen Femurteil 5 und Tibiateil 1 möglich ist, wohingegen in der Flexion ein gewisses Mass an Rotation möglich ist. Folglich eignet sich die Kniegelenkprothese insbesondere als Implantat für Patienten, bei denen sowohl die Kreuzbänder als auch die Seitenbänder entweder nicht mehr vorhanden oder nicht mehr funktionstüchtig sind.

In Fig. 3 erkennt man eine Explosionsdarstellung des als Kupplungsorgan dienenden Zapfens 4, des Lagerkörpers 3, und von verschiedenen Ausführungsbeispielen von Zapfen 2,2a,2b,2c,2d,2e, die als Führungsstück dienen. Fig. 4 zeigt diese als Führungstücke dienenden Zapfen oder Teile davon in einer perspektivischen Darstellung von schräg unten.

Bei dem in Fig. 3 ganz links angeordneten Ausführungsbeispiel des Zapfens 2 handelt es sich um das bereits anhand von Fig. 2 erläuterte Ausführungsbeispiel. Die drehfeste Anordnung des Zapfens 2 beruht auf der ovalen Ausbildung des unteren Bereichs 20, der in einen entsprechend geformten Bereich der Bohrung 13 (Fig. 2) eingreift. Die ovale Ausbildung des Bereichs 20 ekennt man noch besser aus Fig. 4, wo dieses Ausführungsbeispiel des Zapfens 2 ebenfalls ganz links (obere Zeile) angeordnet ist. Auf die Darstellung des oberen Bereichs 22 des Zapfens 2 ist dabei verzichtet worden.

Bei dem in Fig. 3 als zweites von links angeordneten Ausführungsbeispiel erfolgt die drehfeste Anordnung des Zapfens 2a derart, dass der mittlere Bereich 21a und der zugehörige Bereich der Bohrung im Tibiateil 1 kreiszylindrisch ausgebildet sind. Der untere Bereich 20a ist zwar ebenfalls kreiszylindrisch ausgebildet, jedoch axial versetzt in Bezug auf die Längsachse des mittleren Bereichs 21a, was noch besser aus Fig. 4 zu erkennen ist, wo der mittlere Bereich 21a und der untere Bereich 20a gezeigt sind (zweites Beispiel von links, obere Zeile). Die beiden Bereiche 20a und 21a können also in dem jeweils zugehörigen Abschnitt der Bohrung 13 nur um unterschiedliche Achsen gedreht werden, was bei einer versuchten Drehung des Zapfens 2a sofort zu einer Blockierung führt.

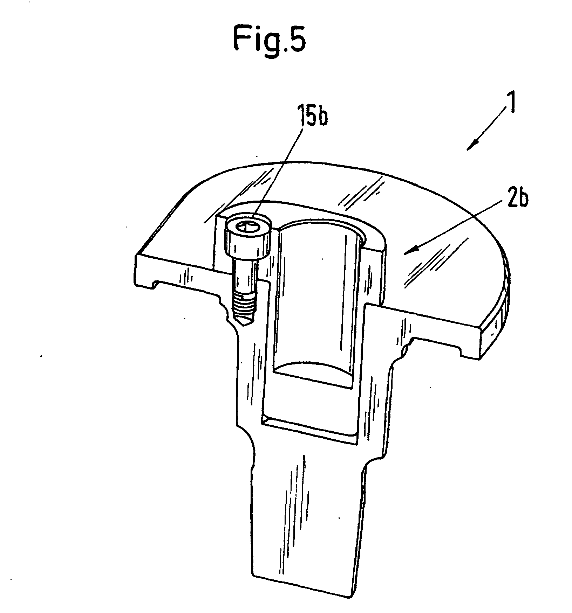

Bei dem in Fig. 3 als drittes von links angeordneten Ausführungsbeispiel erfolgt die drehfeste Sicherung des Zapfens 2b mittels einer separaten Verankerung des Zapfens 2b mit Hilfe einer Verschraubung. Dazu weist das Tibiateil 1 allerdings eine zusätzliche, als Gewindesackloch ausgebildete Bohrung 15b (Fig. 5) auf. Der untere Bereich und der mittlere Bereich des Zapfens 2b sind zu einem gemeinsamen, kreiszylindrischen unteren Bereich 20a zuammengeführt worden (siehe auch Fig. 4, zweites Beispiel von rechts in der oberen Zeile), weil ja die Fixierung nicht über die geometrische Gestalt dieser Bereiche erfolgt. Dieses Ausführungsbeispiel ist in der Fig. 5 noch einmal in einer Darstellung in bereits zusammengesetztem und fixiertem Zustand gezeigt.

Bei dem in Fig. 3 als drittes von rechts angeordneten Ausführungsbeispiel erfolgt die drehfeste Anordnung des Zapfens 2c durch einen sich in Längsrichtung des Zapfens erstreckenden Vorsprung 20c und durch beidseits links und rechts dieses Vorsprungs 20c angeordnete, in Längsrichtung des Zapfens verlaufende Nuten 21c (siehe auch Fig. 4, Beispiel ganz rechts in der oberen Zeile). Der untere und mittlere Bereich des Zapfens 2c sind auch hier wieder zu einem Bereich zusammengeführt worden.

Die beiden Ausführungsbeispiele 2d und 2e, die in Fig. 3 rechts dargestellt sind, scheinen auf den ersten Blick keinen Unterschied aufzuweisen. Betrachtet man aber die untere Zeile in Fig. 4, so erkennt man, dass im Falle des Zapfens 2d dieser an seinem oberen Bereich 22d einen separaten Zapfen 21d aufweist. Alternativ könnte anstelle des separaten Zapfens 21d auch im oberen Bereich 22d eine als Sackloch ausgebildete Bohrung vorgesehen sein, in der ein separat herstellbarer Stift fixiert (z.B. eingepresst) werden kann. Bei diesem Ausführungsbeispiel (Fig. 4, untere Zeile links) ist dann in dem Tibiateil 1 eine entsprechende Bohrung vorgesehen, die diesen Zapfen 21d (oder den Stift) aufnimmt. Dadurch ist auch in diesem Fall der als Führungsstück dienende Zapfen 2d drehfest in Bezug auf das Tibiateil 1 angeordnet.

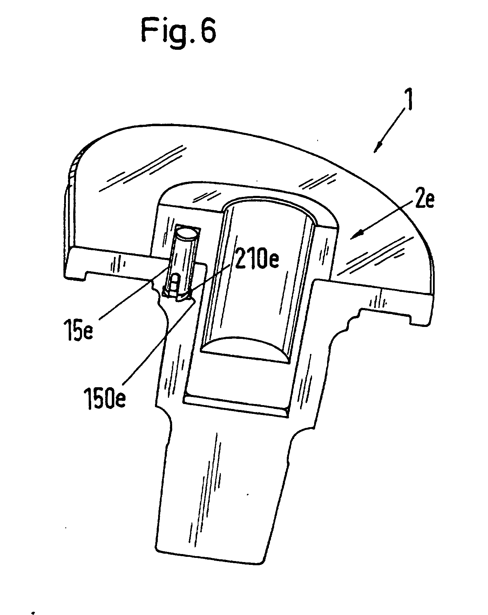

Bei dem Zapfen 2e (Fig. 4, untere Zeile rechts) sind anstelle des separaten Zapfens zwei federnde Elemente 21e mit Vorsprüngen 210e vorgesehen, die in eine entsprechende separate als Sackloch ausgebildete Bohrung 15e im Tibiateil 1 eingeführt werden. Die separate Bohrung 15e weist eine Hinterschneidung 150e auf, in die die Vorsprünge 210e der federnden Elemente 21e eingreifen. Ein solches Ausführungsbeispiel ist in Fig. 6 noch einmal in einer Darstellung in zusammengesetztem Zustand gezeigt. Die federnden Elemente 21e können dabei direkt am Zapfen 2e angeformt sein (also einen integralen Bestandteil des Zapfens bilden), wie in Fig. 4 zu erkennen (untere Zeile rechts). Es kann aber auch eine entsprechende als Sackloch ausgebildete Bohrung im oberen Bereich 22e des Zapfens 2e vorgesehen sein, in der ein separat herstellbarer Stift mit entsprechenden federnden Elementen fixiert werden kann, wie es in Fig. 6 gezeigt ist. Man erkennt, dass hier noch viele weitere Varianten der drehfesten Anordnung des als Führungsstück dienenden Zapfens möglich sind, die hier diskutierten sind daher als beispielhaft zu betrachten.

Schliesslich soll noch angemerkt werden, dass das Tibiateil 1, der als Führungsstück ausgebildete Zapfen 2, der Zapfen 4 wie auch das Femurteil 5 typischerweise aus einem Metall oder einer Metallegierung hergestellt sind, wie sie üblicherweise bei Gelenkprothesen verwendet werden, während der Lagerkörper 3 typischerweise aus einem Kuststoff (z.B. Polyethylen) hergestellt ist.

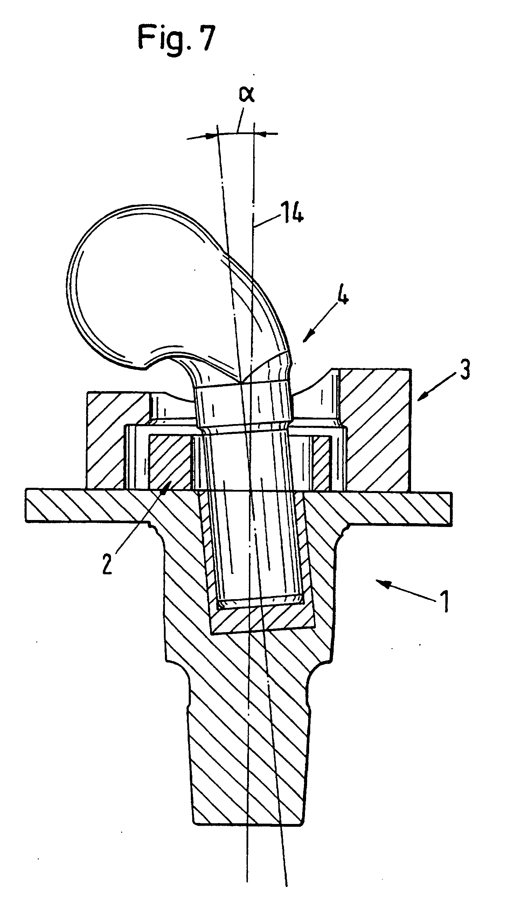

Fig. 7 schliesslich zeigt ein weiteres Ausführungsbeispiel einer erfindungsgemässen Kniegelenkprothese (in Schnittdarstellung), wobei das Femurteil 5 nicht dargestellt ist. Der wesentliche Unterschied zu den vorher erläuterten Varianten besteht darin, dass der Zapfen 4 hier um einen Winkel α zur Längsachse 14 (oder einer hierzu parallelen Achse) geneigt angeordnet ist. Typischerweise beträgt dieser Winkel α nur einige wenige Grad, z.B. bis etwa 15 Grad. Ansonsten kann die Kniegelenkprothese vom Prinzip her gleich aufgebaut sein wie die anhand von Fig. 2 bis Fig. 6 erläuterte Kniegelenkprothese.

Zusammenfassend umfasst die Kniegelenkrothese ein Tibiateil, welches eine Tibialagerfläche aufweist, sowie einen Lagerkörper, der auf der Tibialagerfläche gleitend verschiebbar gelagert ist und der auf seiner der Tibialagerfläche abgewandten Seite Lagerschalen aufweist. Ferner umfasst sie ein Femurteil, welches auf den Lagerschalen des Lagerkörpers beweglich angeordnet ist, sowie ein relativ zum Tibiateil drehfest angeordnetes Führungsstück, welches lediglich eine Verschiebung des Lagerkörpers in sagittaler Richtung relativ zur Tibialagerfläche gestattet. Weiterhin umfasst sie ein Kupplungsorgan, welches sowohl mit dem Führungsstück als auch mit dem Femurteil zusammenwirkt und eine Drehung des Femurteils auf dem Lagerkörper erlaubt. Das Kupplungsorgan weist einen in Richtung der Längsachse des Tibiateils bzw. einer hierzu parallelen Drehachse oder in Richtung einer relativ zur Längsachse des Tibiateils bzw. einer hierzu parallelen Achse geneigt sich erstreckenden, drehbar gelagerten, Zapfen auf.

## Patentansprüche

1. Kniegelenkprothese,
mit einem Tibiateil (1), welches eine Tibialagerfläche (10) aufweist,
mit einem Lagerkörper (3), der auf der Tibialagerfläche (10) gleitend verschiebbar gelagert ist und der auf seiner der Tibialagerfläche (10) abgewandten Seite Lagerschalen (31) aufweist,
mit einem Femurteil (5), welches auf den Lagerschalen (31) des Lagerkörpers (3) beweglich angeordnet ist,
sowie mit einem relativ zum Tibiateil (1) drehfest angeordneten Führungstück (2), welches lediglich eine Verschiebung des Lagerkörpers (3) in sagittaler Richtung relativ zur Tibialagerfläche (1) gestattet,
und mit einem Kupplungsorgan, welches sowohl mit dem Führungsstück (2) als auch mit dem Femurteil (5) zusammenwirkt und eine Drehung des Femurteils (5) auf dem Lagerkörper (3) erlaubt,
wobei das Kupplungsorgan einen im Führungsstück (2) drehbar gelagerten Zapfen (4) aufweist, der sich in Richtung der Längsachse (14) des Tibiateils (1) bzw. einer hierzu parallelen Drehachse erstreckt oder in Richtung einer relativ zur Längsachse (14) des Tibiateils ( 1 ) bzw. einer hierzu parallelen Drehachse geneigten Achse,
wobei zur Varus/Valgus-Stabilisierung des Femurteils (5) relativ zum Tibiateil (1) der als Kupplungsorgan dienende Zapfen (4) an seinem dem Femurteil (5) zugewandten Ende ein Stabilisierungsstück (41) mit Seitenwänden (410) und das Femurteil (5) entsprechende mit den Seitenwänden (410) des Stabilisierungsstücks (41) zusammenwirkende Stabilisierungsflächen (510) aufweist, und
wobei die Stabilisierungsflächen (510) des Femurteils (5) gegenüberliegende Innenseiten eines Stabilisierungskastens (51) des Femurteils (5) bilden, in den das Stabilisierungsstück (41) zur Koppelung des Tibiateils (1) und des Femurteils (5) beim Zusammensetzen der Prothese hineingleiten kann.

2. Kniegelenkprothese nach Anspruch 1, wobei das Führungsstück (2) am Tibiateil (1) angeformt ist.

3. Kniegelenkprothese nach Anspruch 1, wobei sowohl das Führungsstück als auch das Kupplungsorgan beide als separate Zapfen (2,4) ausgebildet sind, wobei, sowohl der als Führungsstück dienende Zapfen (2) als auch das Tibiateil (1) Mittel zur drehfesten Anordnung des als Führungsstück (2) dienenden Zapfens im Tibiateil (1) aufweisen.

4. Kniegelenkprothese nach einem der vorangehenden Ansprüche, wobei das Tibiateil (1) einen Verankerungsabschnitt (11) aufweist, der mit einem separaten Verankerungsschaft (12) verbindbar ist.

5. Kniegelenkprothese nach einem der vorangehenden Ansprüche, wobei das Femurteil (5) einen Verankerungsabschnitt (52) aufweist, der mit einem separaten Verankerungsschaft (53) verbindbar ist.

## Claims

1. A knee joint prosthesis,
comprising a tibial part (1) which has a tibial support surface (10);
comprising a support body (3) which is slidingly displaceably supported on the tibial support surface (10) and which has support shells (31) at its side remote from the tibial support surface (10);
comprising a femoral part (5) which is movably arranged on the support shells (31) of the support body (3);
and comprising a guide piece (2) which is rotationally fixedly arranged relative to the tibial part (1) and which only allows a displacement of the bearing body (3) in the sagittal direction relative to the tibial support surface (1);
and comprising a coupling member which cooperates both with the guide piece (2) and with the femoral part (5) and allows a rotation of the femoral part (5) on the bearing body (3),
wherein the coupling member has a spigot (4) which is rotatably supported in the guide piece (2) and which extends in the direction of the longitudinal axis (14) of the tibial part (1) or in the direction of a rotational axis parallel thereto or in the direction of an axis inclined relative to the longitudinal axis (14) of the tibial part (1) or in the direction of an axis inclined relative to a rotational axis parallel thereto,
wherein the spigot (4) serving as a coupling member has a stabilisation piece (41) with side walls (410) at its end facing the femoral part (5) and the femoral part (5) has corresponding stabilisation surfaces (510) cooperating with the side walls (410) of the stabilisation piece (41) for the varus/valgus stabilisation of the femoral part (5) relative to the tibial part (1), and
wherein the stabilisation surfaces (510) of the femoral part (5) form oppositely disposed internal sides of a stabilisation box (51) of the femoral part (5) into which the stabilisation piece (41) can slide for the coupling of the tibial part (1) and of the femoral part (5) on the assembly of the prosthesis.

2. A knee joint prosthesis in accordance with claim 1, wherein the guide piece (2) is moulded to the tibial part (1).

3. A knee joint prosthesis in accordance with claim 1, wherein both the guide piece and the coupling member are both made as separate spigots (2, 4), with both the spigot (2) serving as the guide piece and the tibial part (1) having means for the rotationally fixed arrangement of the spigot serving as the guide piece (2) in the tibial part (1).

4. A knee joint prosthesis in accordance with any one of the preceding claims, wherein the tibial part (1) has an anchoring section (11) which can be connected to a separate anchoring shaft (12).

5. A knee joint prosthesis in accordance with any one of the preceding claims, wherein the femoral part (5) has an anchoring section (52) which can be connected to a separate anchoring shaft (53).

## Revendications

1. Prothèse de l'articulation du genou,
comportant une partie tibiale (1), qui présente une surface d'appui tibiale (10) ;
un corps d'appui (3), qui est monté sur la surface d'appui tibiale (10) de manière mobile par glissement et qui présente, sur son côté dirigé à l'opposé de la surface d'appui tibiale (10), des coques d'appui (31) ;
une partie fémorale (5), qui est disposée de manière mobile sur les coques d'appui (31) du corps d'appui (3) ;
ainsi qu'une pièce de guidage (2), placée d'une façon antitorsion par rapport à la partie tibiale (1), et qui autorise uniquement un déplacement du corps d'appui (3) dans le sens sagittal par rapport à la surface d'appui tibiale (1) ;
et un organe d'accouplement, qui coopère aussi bien avec la pièce de guidage (2) qu'avec la partie fémorale (5) et qui permet une rotation de la partie fémorale (5) sur le corps d'appui (3),
dans laquelle l'organe d'accouplement présente un pivot (4), qui est monté de manière à pouvoir tourner dans la pièce de guidage (2), et qui s'étend dans le sens de l'axe longitudinal (14) de la partie tibiale (1) ou dans celui d'un axe de rotation parallèle à celle-ci, ou bien encore dans le sens d'un axe, qui est incliné par rapport à l'axe longitudinal (14) de la partie tibiale (1), ou par rapport à un axe de rotation parallèle à celle-ci,
dans laquelle, pour une stabilisation varus / valgus de la partie fémorale (5) par rapport à la partie tibiale (1), le pivot (4) servant d'organe d'accouplement présente, au niveau de son extrémité dirigée vers la partie fémorale (5), une pièce de stabilisation (41) comportant des parois latérales (410), tandis que la partie fémorale (5) présente des surfaces correspondantes de stabilisation (510), qui coopèrent avec les parois latérales (410) de la pièce de stabilisation (41), et
dans laquelle les surfaces de stabilisation (510) de la partie fémorale (5) constituent des faces internes opposées d'un logement de stabilisation (51) de la partie fémorale (5), dans lequel peut pénétrer par glissement la pièce de stabilisation (41) afin de relier la partie tibiale (1) et la partie fémorale (5) lors de l'assemblage de la prothèse.

2. Prothèse de l'articulation du genou selon la revendication 1, dans laquelle la pièce de guidage (2) est formée d'un seul tenant sur la partie tibiale (1).

3. Prothèse de l'articulation du genou selon la revendication 1, dans laquelle aussi bien la pièce de guidage que l'organe d'accouplement sont réalisés tous les deux en tant que pivots (2, 4) distincts, et dans laquelle aussi bien le pivot (2) servant de pièce de guidage que la partie tibiale (1) présentent des moyens pour une mise en place antitorsion du pivot servant de pièce de guidage (2) dans la partie tibiale (1).

4. Prothèse de l'articulation du genou selon l'une des revendications précédentes, dans laquelle la partie tibiale (1) présente une partie d'ancrage (11), qui peut être reliée à une tige d'ancrage (12) séparée.

5. Prothèse de l'articulation du genou selon l'une des revendications précédentes, dans laquelle la partie fémorale (5) présente une partie d'ancrage (52), qui peut être reliée à une tige d'ancrage (53) séparée.
